# EUROPEAN PATENT APPLICATION

(11) **EP 4 438 148 A1**
(43) Date of publication of application: **02.10.2024**
(21) Application number: 24158897.9
(22) Date of filing: 21.02.2024
(51) Int. Cl.: B01B 1/06

(54) **ULTRASOUND PROBE**

(30) Priority: 29.03.2023 JP 2023053131
(71) Applicant: FUJIFILM Corporation, Tokyo 106-8620 (JP)
(72) Inventor: HASE, Takatoshi, Kanagawa (JP)
(74) Representative: Dehns Germany Partnerschaft mbB

(57) **Abstract**

Provided is an ultrasound probe capable of suppressing crosstalk between a plurality of divided element portions.

An ultrasound probe includes: a plurality of piezoelectric elements (2) that are arranged on a backing material (1) along an azimuth direction, in which each of the plurality of piezoelectric elements consists of a laminate in which a signal electrode layer (2A), a piezoelectric body portion (2B), and a ground electrode layer (2C) are laminated, each of the piezoelectric elements is divided into a plurality of divided element portions (A) in an elevation direction, an acoustic matching layer (3) that is disposed on each of the ground electrode layer is provided, gaps between the plurality of divided element portions are filled with a filler (4), a conductive member (5) is disposed on each of a pair of side surfaces at both ends of each of the divided element portions in the elevation direction, and the conductive member is connected to the ground electrode layer.

## Description

### BACKGROUND OF THE INVENTION

### 1. Field of the Invention

The present invention relates to an ultrasound probe having high sensitivity.

### 2. Description of the Related Art

In the related art, in a medical field, an ultrasound diagnostic apparatus using an ultrasound image has been put into practical use. In general, an ultrasound diagnostic apparatus of this kind transmits an ultrasound beam from an ultrasound probe toward a subject and receives an ultrasound echo from the subject by the ultrasound probe, and an ultrasound image is generated by electrically processing a reception signal thereof.

The ultrasound probe usually has a plurality of piezoelectric elements arranged in an array in a so-called azimuth direction. In general, in the ultrasound probe, the image quality of the ultrasound image may be deteriorated due to so-called crosstalk between a plurality of piezoelectric elements. Therefore, in order to reduce the crosstalk between the plurality of piezoelectric elements, an ultrasound probe having a so-called one-dimensional transducer array in which grooves between the plurality of piezoelectric elements are filled with an insulating member has been developed as disclosed in JP1998-285695A (JP-H10-285695A).

A so-called matrix-shaped transducer array in which a plurality of piezoelectric elements are divided into a plurality of divided element portions in a so-called elevation direction in addition to the azimuth direction is also known. In the matrix-shaped transducer array, a ground electrode layer common to the plurality of piezoelectric elements is often formed. In a case where the piezoelectric element is divided into a plurality of divided element portions for each ground electrode layer in order to suppress crosstalk between the divided element portions of the matrix-shaped transducer array based on the technique in JP1998-285695A (JP-H10-285695A), there are problems that an isolated ground electrode layer is formed and it is difficult to lead a wiring from the ground electrode layer of all the divided element portions.

### SUMMARY OF THE INVENTION

The present invention has been made to solve the above-described problems in the related art, and an object of the present invention is to provide an ultrasound probe in which, in a matrix-shaped transducer array, it is possible to suppress crosstalk between a plurality of divided element portions while leading a wiring from all the ground electrode layers.

According to the following configuration, the above-described object can be achieved.
[1] An ultrasound probe includes a plurality of piezoelectric elements that are arranged in an array on a backing material along an azimuth direction, in which each of the plurality of piezoelectric elements consists of a laminate in which a signal electrode layer, a piezoelectric body portion, and a ground electrode layer are sequentially laminated on a surface of the backing material, each of the piezoelectric elements is divided into a plurality of divided element portions in an elevation direction, a plurality of acoustic matching layers corresponding to the plurality of divided element portions and disposed on the ground electrode layer of each of the divided element portions are provided, gaps between the plurality of piezoelectric elements arranged in the azimuth direction and between the plurality of divided element portions in each of the piezoelectric elements are filled with a filler, a conductive member is disposed on each of a pair of side surfaces at both ends of each of the divided element portions in the elevation direction, and the conductive member is connected to the ground electrode layer of the divided element portion.
[2] The ultrasound probe according to [1], in which the conductive member may extend to a side surface of the acoustic matching layer that is disposed on the ground electrode layer of each of the divided element portions in the elevation direction.
[3] The ultrasound probe according to [1], in which the conductive member formed on the side surface of each of the divided element portions may extend to the divided element portions adjacent along a lower part of the filler that fills gaps between the adjacent divided element portions in the elevation direction, and is connected to the conductive member disposed on the side surface of the adjacent divided element portion.
[4] The ultrasound probe according to any one of [1] to [3], in which the conductive member may have a hardness higher than a hardness of the filler.
[5] The ultrasound probe according to [4], in which the conductive member may have a Shore hardness of 80 or more.
[6] The ultrasound probe according to any one of [1] to [5], in which the conductive member may have a thickness having a dimension smaller than a wavelength of an ultrasonic wave emitted from the piezoelectric element.
[7] The ultrasound probe according to [6], in which the conductive member may have a thickness having a dimension smaller than one-tenth of the wavelength of the ultrasonic wave emitted from the piezoelectric element.
[8] A method of manufacturing an ultrasound probe including a plurality of piezoelectric elements that are arranged in an array on a backing material along an azimuth direction, the method includes forming a laminate in which a first conductive layer, a piezoelectric layer, and a second conductive layer are sequentially laminated on a surface of the backing material; forming an acoustic matching layer on the laminate; forming the plurality of piezoelectric elements arranged along the azimuth direction by dicing the laminate and the acoustic matching layer; dividing each of the piezoelectric elements into a plurality of divided element portions in an elevation direction by dicing each of the piezoelectric elements; disposing a conductive member on a pair of side surfaces at both ends of each of the divided element portions in the elevation direction such that the conductive member is connected to a ground electrode layer consisting of the second conductive layer in each of divided element portions; and filling gaps between the plurality of piezoelectric elements arranged in the azimuth direction and between the plurality of divided element portions in each of the piezoelectric elements with a filler.

According to the present invention, a plurality of piezoelectric elements are arranged in an azimuth direction on a backing material in an array, each of the plurality of piezoelectric elements consists of a laminate in which a signal electrode layer, a piezoelectric body portion, and a ground electrode layer are sequentially laminated on a surface of the backing material, each piezoelectric element is divided into a plurality of divided element portions in an elevation direction, a plurality of acoustic matching layers corresponding to the plurality of divided element portions and disposed on the ground electrode layer of each of the divided element portions are provided, gaps between the plurality of piezoelectric elements arranged in the azimuth direction and between the plurality of divided element portions in each of the piezoelectric elements are filled with a filler, a conductive member is disposed on each of a pair of side surfaces at both ends of each of the divided element portions in the elevation direction, the conductive member is connected to the ground electrode layer of the divided element portion. Therefore, in a matrix-shaped transducer array, it is possible to suppress crosstalk between the plurality of divided element portions while leading a wiring from all the ground electrode layers.

### BRIEF DESCRIPTION OF THE DRAWINGS

Fig. 1 is a perspective view showing a part of an ultrasound probe according to an embodiment of the present invention.
Fig. 2 is a cross-sectional view of the ultrasound probe according to the embodiment of the present invention.
Fig. 3 is a flowchart showing a method of manufacturing of the ultrasound probe according to the embodiment of the present invention.
Fig. 4 is a cross-sectional view of a laminate and an acoustic matching layer formed on a backing material.
Fig. 5 is a cross-sectional view of a plurality of divided element portions and the acoustic matching layer.
Fig. 6 is a cross-sectional view showing a conductive member formed between the plurality of divided element portions.
Fig. 7 is a diagram showing the conductive member that has been diced.
Fig. 8 is a cross-sectional view of an ultrasound probe according to a first modification example of the embodiment of the present invention.
Fig. 9 is a cross-sectional view of an ultrasound probe according to a second modification example of the embodiment of the present invention.
Fig. 10 is a cross-sectional view of an ultrasound probe according to a third modification example of the embodiment of the present invention.
Fig. 11 is a cross-sectional view of an ultrasound probe according to a fourth modification example of the embodiment of the present invention.

### DESCRIPTION OF THE PREFERRED EMBODIMENTS

Hereinafter, embodiments of the present invention will be described with reference to the accompanying drawings.

The description of components described below is provided based on a representative embodiment of the present invention, but the present invention is not limited to such an embodiment.

In the present specification, a numerical range represented using "to" means a range including numerical values before and after "to" as a lower limit value and an upper limit value.

In the present specification, "same" and "identical" include an error range generally allowed in the technical field.

### Embodiment

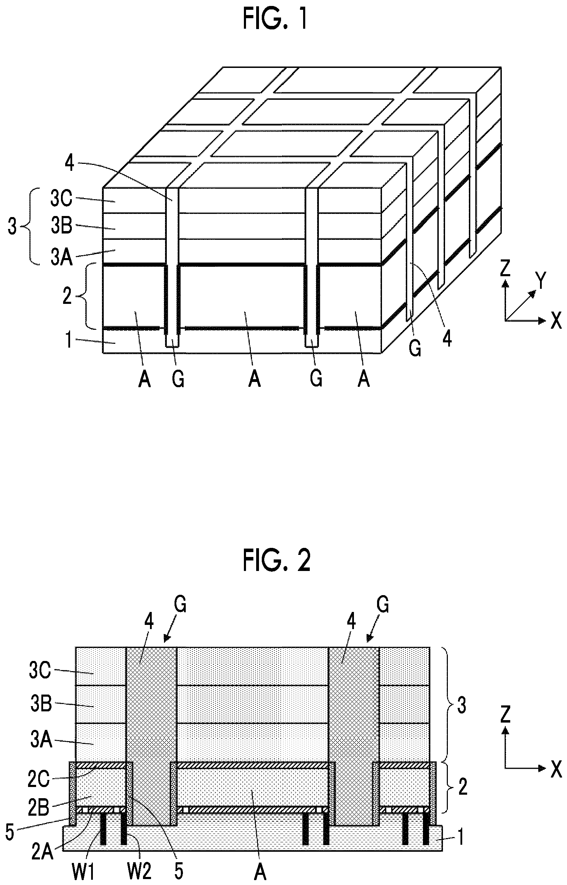

Fig. 1 shows a configuration of an ultrasound probe according to an embodiment of the present invention. The ultrasound probe comprises a backing material 1, a plurality of piezoelectric elements 2 formed on the backing material 1, and an acoustic matching layer 3 formed on the plurality of piezoelectric elements 2.

The plurality of piezoelectric elements 2 are arranged in an array along a Y direction, that is, an azimuth direction. A groove G is formed between the plurality of piezoelectric elements 2, and the groove G is filled with a filler 4. In addition, the plurality of piezoelectric elements 2 are divided into a plurality of divided element portions A in an X direction, that is, an elevation direction by the groove G, and the groove G is filled with the filler 4. Therefore, the plurality of divided element portions A are arranged in the X direction and the Y direction to form a matrix-shaped transducer array.

The acoustic matching layer 3 is configured with a laminate in which a first acoustic matching layer 3A, a second acoustic matching layer 3B, and a third acoustic matching layer 3C are sequentially laminated on the plurality of piezoelectric elements 2. The grooves G dividing the piezoelectric element 2 in the X direction and the Y direction are formed to reach from the third acoustic matching layer 3C to the middle of the backing material 1. Therefore, the acoustic matching layer 3 is also divided into a plurality of portions in the X direction and the Y direction in the same manner as the piezoelectric element 2.

In the following, a height direction of the ultrasound probe that is orthogonal to the X direction and the Y direction is referred to as a Z direction.

The backing material 1 supports the plurality of piezoelectric elements 2 and absorbs the ultrasonic waves released rearward, and is formed of a rubber material such as ferrite rubber.

The acoustic matching layer 3 is a layer for matching the acoustic impedance between the piezoelectric element 2 and a subject to make it easy to incident ultrasonic waves into the subject. The acoustic matching layer 3 can be formed of a material having an acoustic impedance that is a value lower than the acoustic impedance of the piezoelectric element 2 and higher than the acoustic impedance of the subject. For example, by setting the acoustic impedance of the first acoustic matching layer 3A to be smaller than the acoustic impedance of the second acoustic matching layer 3B and by setting the acoustic impedance of the second acoustic matching layer 3B to be smaller than the acoustic impedance of the third acoustic matching layer 3C, a layer structure in which the acoustic impedance gradually decreases from the piezoelectric element 2 toward the subject is formed.

The filler 4 is for fixing the positions and postures of the adjacent piezoelectric element 2 in the Y direction and the adjacent divided element portion A and the acoustic matching layer 3 in the Z direction, and is formed of, for example, an epoxy resin. As the filler 4, more specifically, silicone rubber LSR 2630 (manufactured by MOMENTIVE performance materials Inc., 32 of Shore A hardness), silicone rubber LSR 2030 (manufactured by MOMENTIVE performance materials Inc., 31 of Shore A hardness), silicone rubber RTV 615 (manufactured by MOMENTIVE performance materials Inc., 44 of Shore A hardness), or the like can be used.

As illustrated in Fig. 2, the piezoelectric element 2 consists of a laminate in which a signal electrode layer 2A, a piezoelectric body portion 2B, and a ground electrode layer 2C are sequentially laminated on a surface of the backing material 1.

The piezoelectric body portion 2B is formed of a known piezoelectric material, expands and contracts with an application of a voltage, and expands and contracts with an application of so-called ultrasound echo from the outside to generate an electric signal. The piezoelectric material is, for example, made of a piezoelectric ceramics, such as lead zirconate titanate (PZT), or a polymer material, such as a polyvinylidene fluoride (PVDF).

The signal electrode layer 2A is used for applying a voltage to the piezoelectric body portion 2B and for extracting an electric signal generated by the expanding and contracting of the piezoelectric body portion 2B due to the propagation of the so-called ultrasound echo. A signal wiring W1 for applying a voltage and extracting an electric signal is led out from the signal electrode layer 2A. The signal wiring W1 extends from the signal electrode layer 2A through the backing material 1 to the outside of the ultrasound probe.

The ground electrode layer 2C is used to set a reference potential of the piezoelectric body portion 2B to a ground potential.

The conductive member 5 is disposed on a pair of side surfaces at both ends of the divided element portion A of the piezoelectric element 2 in the X direction. The conductive member 5 extends from the ground electrode layer 2C to the surface of the backing material 1 and is electrically connected to an end portion of the ground electrode layer 2C in the X direction. In addition, a ground wiring W2 for making a potential of the ground electrode layer 2C a ground potential is connected to the conductive member 5. The ground wiring W2 extends from the conductive member 5 to the outside of the ultrasound probe through the backing material 1.

In general, in a matrix-shaped transducer array in which a plurality of piezoelectric elements 2 are divided in the X direction and the Y direction, a continuous ground electrode layer 2C that is formed by being adhered to a surface of the plurality of piezoelectric body portions 2B on the acoustic matching layer 3 side is often formed, and a continuous acoustic matching layer 3 that is formed to cross over the plurality of piezoelectric body portions 2B is often formed on the ground electrode layer 2C. In this case, the vibration of the plurality of piezoelectric body portions 2B or the vibration of the ultrasound echo propagating from the outside may propagate through the acoustic matching layer 3, and thus so-called crosstalk may occur between the plurality of piezoelectric body portions 2B.

Therefore, in a case of attempting to divide the acoustic matching layer 3 in the same manner as the plurality of piezoelectric elements 2 in order to suppress the crosstalk between the plurality of piezoelectric body portions 2B, the ground electrode layer 2C is also divided, so that a plurality of isolated ground electrode layers 2C are formed, and there is a problem that it is difficult to lead a wiring from each of the ground electrode layers 2C.

In the ultrasound probe according to the embodiment of the present invention, the plurality of piezoelectric elements 2 are divided into a plurality of divided element portions A in the X direction, the acoustic matching layer 3 is disposed on the ground electrode layer 2C for each of the plurality of divided element portions A, the conductive member 5 is disposed on each of a pair of side surfaces at both ends of each of the divided element portions A in the X direction, and the conductive member 5 is connected to the ground electrode layer 2C of the divided element portion A. Therefore, it is possible to suppress crosstalk between the plurality of divided element portions Awhile leading each of the ground wirings W2 from all the plurality of ground electrode layers 2C via the conductive member 5.

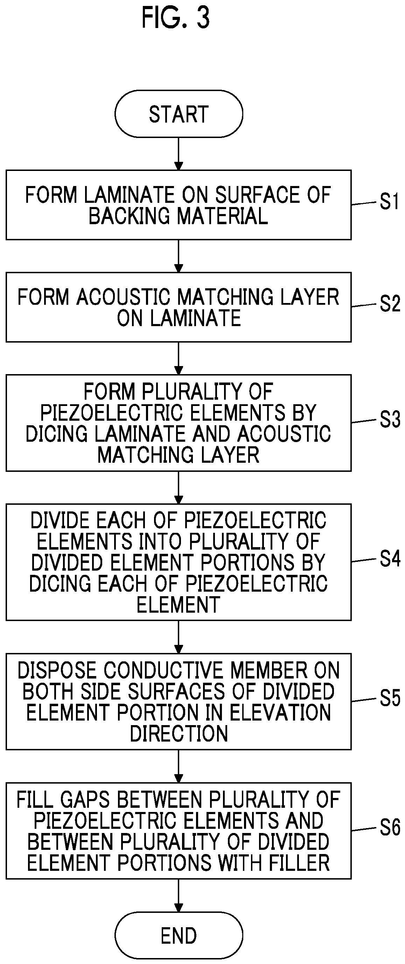

Next, a method of manufacturing of the ultrasound probe according to the embodiment of the present invention will be described referring to a flowchart of Fig. 3.

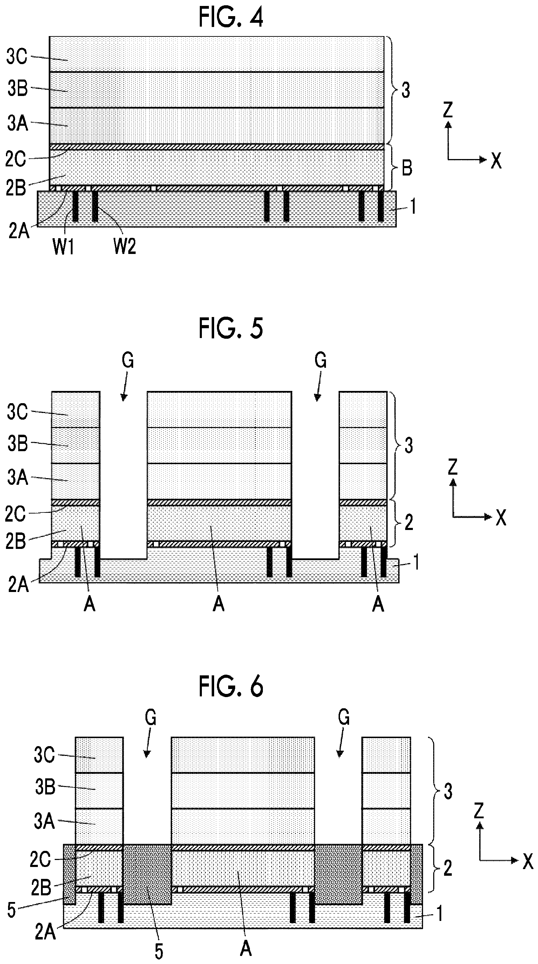

First, in step S1, as shown in Fig. 4, the laminate B in which the signal electrode layer 2A, the piezoelectric body portion 2B, and the ground electrode layer 2C are sequentially laminated on the surface of the backing material 1 is formed by bonding the laminate B with an adhesive or the like.

In step S2, the first acoustic matching layer 3A, the second acoustic matching layer 3B, and the third acoustic matching layer 3C are sequentially bonded onto the laminate B at a temperature of, for example, 80°C to 100°C to form the acoustic matching layer 3.

In step S3, the laminate B and the acoustic matching layer 3 are diced to be divided into a plurality of portions in the Y direction, thereby forming a plurality of piezoelectric elements 2.

In step S4, as shown in Fig. 5, the plurality of piezoelectric elements 2 are diced to be divided into a plurality of divided element portions A in the X direction. In this case, the plurality of acoustic matching layers 3 arranged in the Y direction are also diced to be divided into a plurality of portions in the X direction.

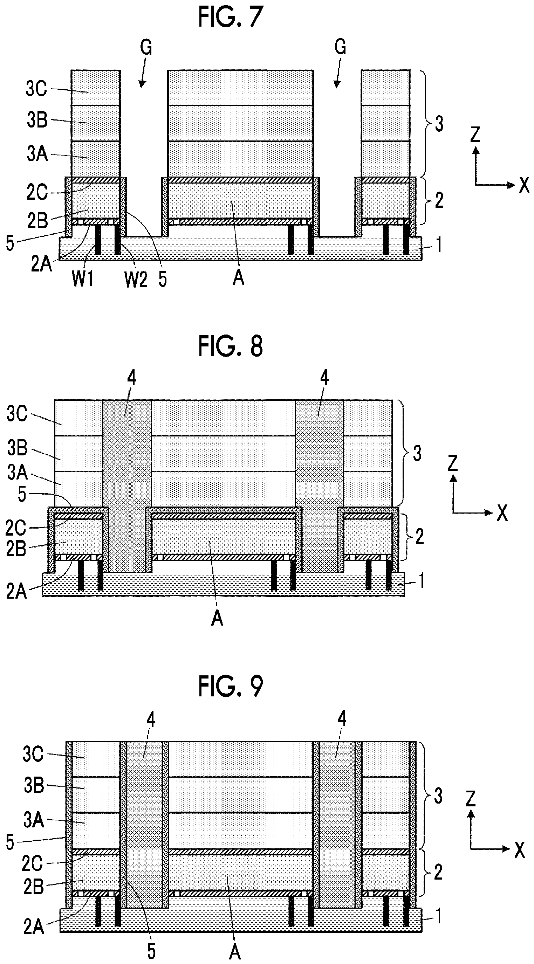

In step S5, the conductive member 5 is disposed on both side surfaces of each of the plurality of divided element portions A in the X direction. In this case, first, as shown in Fig. 6, gaps between the plurality of divided element portions A are filled with the conductive member 5. Thereafter, the conductive member 5 between the divided element portions A is diced to dispose the conductive member 5 on both side surfaces of the divided element portion A as shown in Fig. 7.

Finally, in step S6, gaps between the plurality of divided element portions A in the X direction, between the plurality of acoustic matching layers 3 in the X direction, between the plurality of piezoelectric elements 2 in the Y direction, and between the plurality of acoustic matching layers 3 in the Y direction are filled with the filler 4. Since the positions of the plurality of divided element portions A are fixed by the conductive member 5 disposed on both side surfaces of the plurality of divided element portions A, the laminate consisting of divided element portion A and the acoustic matching layer 3 is prevented from falling down and the ultrasound probe is prevented from being damaged in a case of being filled with the filler 4.

In this manner, the ultrasound probe shown in Figs. 1 and 2 is manufactured by performing steps S1 to S6.

The material of the conductive member 5 is not particularly limited, but for example, a metal or a conductive resin can be used. In addition, in order to prevent the laminate consisting of the divided element portion A and the acoustic matching layer 3 from falling down and to prevent the ultrasound probe from being damaged during the manufacture of the ultrasound probe, the conductive member 5 preferably has a height higher than that of the filler 4, and specifically, the conductive member 5 preferably has a Shore hardness of, for example, 65 or more, and more preferably has a Shore hardness of 80 or more. Examples of a material of the conductive member 5 having such a hardness include silver epoxy EPO-TEK E2101 (manufactured by Epoxy Technology Inc., 68 of Shore D hardness), silver epoxy EPO-TEK H37-MP (manufactured by Epoxy Technology Inc., 80 of Shore D hardness), and silver epoxy EPO-TEK H35-175MP (manufactured by Epoxy Technology Inc., 83 of Shore D hardness).

In addition, in order that the presence of the conductive member 5 does not affect the acoustic performance of the ultrasound probe, the conductive member 5 preferably has a thickness having a dimension smaller than the wavelength of the ultrasonic wave emitted from the piezoelectric element 2, and specifically, the conductive member 5 preferably has a thickness having a dimension smaller than 1/10 of the wavelength of the ultrasonic wave emitted from the piezoelectric element 2.

In addition, the conductive member 5 is described to be disposed on both side surfaces of the divided element portion A in the X direction and is electrically connected to the end portion of the ground electrode layer 2C in the X direction, but the conductive member 5 can also be disposed to extend from one side surface of the divided element portion A to the other side surface of the divided element portion A through an upper surface of the ground electrode layer 2C, for example, as shown in Fig. 8. In this case, since the conductive member 5 is electrically connected to both ends of the ground electrode layer 2C in the X direction and to a surface of the ground electrode layer 2C on the acoustic matching layer 3 side, the reliability of the electrical connection between the conductive member 5 and the ground electrode layer 2C is improved.

In addition, the conductive member 5 is described to extend from the ground electrode layer 2C of the divided element portion A to the surface of the backing material 1, but the conductive member 5 can also be disposed, for example, as shown in Fig. 9, to extend to side surface of the acoustic matching layer 3 in the X direction disposed on the ground electrode layer 2C of the divided element portion A, that is, to extend from the third acoustic matching layer 3C to the surface of the backing material 1. In this case, since the entire laminate consisting of the divided element portion A and the acoustic matching layer 3 is supported by the conductive member 5, for example, the laminate consisting of the divided element portion A and the acoustic matching layer 3 is further prevented from falling down and being damaged during the manufacture of the ultrasound probe.

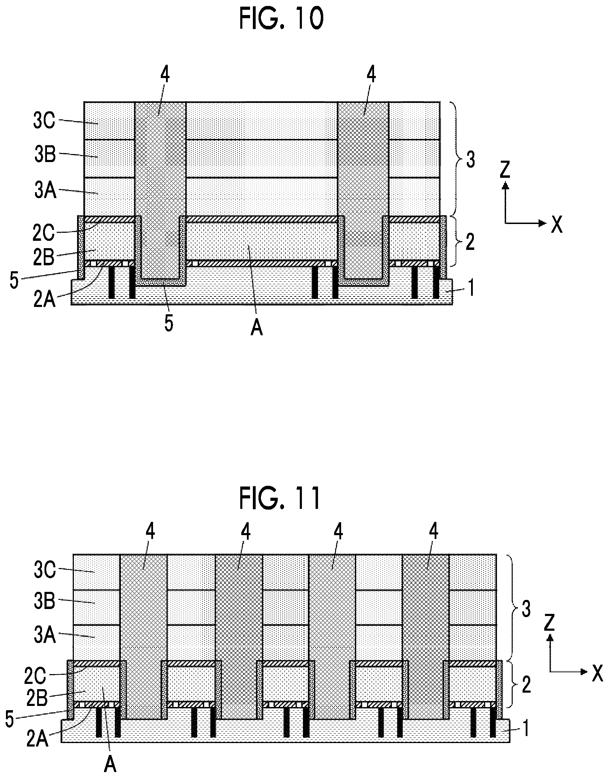

In addition, each of the conductive members 5 is described to be electrically connected to one corresponding ground electrode layer 2C, for example, as shown in Fig. 10, but the conductive member 5 can also be disposed to extend along a lower part of the filler 4 that fills gaps between the divided element portions A adjacent in the X direction, that is, the divided element portions A adjacent along the surface of the backing material 1, and to be connected to the conductive member 5 disposed on the side surface of the adjacent divided element portions A. In this case, since the ground electrode layers 2C of the plurality of divided element portions A are electrically connected to the plurality of conductive members 5, the potential of the ground electrode layers 2C of the plurality of divided element portions A can be stably set to the ground potential.

In addition, the piezoelectric element 2 is divided into three divided element portions A in the X direction, but for example, the piezoelectric element 2 can be divided into two divided element portions A in the X direction, or can be divided into four or more divided element portions A. For example, Fig. 11 shows an ultrasound probe in which the piezoelectric element 2 is divided into five divided element portions A in the X direction. In this manner, by dividing the piezoelectric element 2 into a plurality of divided element portions A in the X direction, it is possible to finely adjust a so-called depth of field in a case where a so-called ultrasound beam is transmitted from the ultrasound probe. In this case, a ratio of the dimension of the divided element portion A in the Z direction to the dimension of the divided element portion A in the X direction is increased. However, since both side surfaces of the divided element portion A in the X direction are supported by the conductive member 5, the laminate consisting of the divided element portion A and the acoustic matching layer 3 is prevented from falling down and being damaged during the manufacture of the ultrasound probe.

In addition, the acoustic matching layer 3 is described to be composed of three layers of the first acoustic matching layer 3A, the second acoustic matching layer 3B, and the third acoustic matching layer 3C, but the acoustic matching layer 3 can also be composed of two or less layers or can also be composed of four or more layers.

### Explanation of References

1: backing material
2: piezoelectric element
2A: signal electrode layer
2B: piezoelectric body portion
2C: ground electrode layer
4: filler
5: conductive member
A: divided element portion
B: laminate
G: groove
W1: signal wiring
W2: ground wiring

## Claims

1. An ultrasound probe comprising:
a plurality of piezoelectric elements (2) that are arranged in an array on a backing material (1) along an azimuth direction,
wherein each of the plurality of piezoelectric elements (2) consists of a laminate (B) in which a signal electrode layer (2A), a piezoelectric body portion (2B), and a ground electrode layer (2C) are sequentially laminated on a surface of the backing material (1),
each of the piezoelectric elements (2) is divided into a plurality of divided element portions (A) in an elevation direction,
a plurality of acoustic matching layers (3) corresponding to the plurality of divided element portions (A) and disposed on the ground electrode layer (2C) of each of the divided element portions (A) are provided,
gaps between the plurality of piezoelectric elements (2) arranged in the azimuth direction and between the plurality of divided element portions (A) in each of the piezoelectric elements (2) are filled with a filler (4),
a conductive member (5) is disposed on each of a pair of side surfaces at both ends of each of the divided element portions (A) in the elevation direction, and
the conductive member (5) is connected to the ground electrode layer (2C) of the divided element portion (A).

2. The ultrasound probe according to claim 1,
wherein the conductive member (5) extends to a side surface of the acoustic matching layer (3) that is disposed on the ground electrode layer (2C) of each of the divided element portions (A) in the elevation direction.

3. The ultrasound probe according to claim 1,
wherein the conductive member (5) formed on the side surface of each of the divided element portions (A) extends to the divided element portions adjacent along a lower part of the filler (4) that fills gaps between the adjacent divided element portions (A) in the elevation direction, and is connected to the conductive member (5) disposed on the side surface of the adjacent divided element portion (A).

4. The ultrasound probe according to any one of claims 1 to 3,
wherein the conductive member (5) has a hardness higher than a hardness of the filler (4).

5. The ultrasound probe according to claim 4,
wherein the conductive member (5) has a Shore hardness of 80 or more.

6. The ultrasound probe according to any one of claims 1 to 5,
wherein the conductive member (5) has a thickness having a dimension smaller than a wavelength of an ultrasonic wave emitted from the piezoelectric element (2).

7. The ultrasound probe according to claim 6,
wherein the conductive member (5) has a thickness having a dimension smaller than one-tenth of the wavelength of the ultrasonic wave emitted from the piezoelectric element (2).

8. A method of manufacturing an ultrasound probe including a plurality of piezoelectric elements (2) that are arranged in an array on a backing material (1) along an azimuth direction, the method comprising:
forming a laminate (B) in which a first conductive layer, a piezoelectric layer (2B), and a second conductive layer are sequentially laminated on a surface of the backing material (1);
forming an acoustic matching layer (3) on the laminate (B);
forming the plurality of piezoelectric elements (2) arranged along the azimuth direction by dicing the laminate (B) and the acoustic matching layer (3);
dividing each of the piezoelectric elements (2) into a plurality of divided element portions (A) in an elevation direction by dicing each of the piezoelectric elements (2);
disposing a conductive member (5) on a pair of side surfaces at both ends of each of the divided element portions (A) in the elevation direction such that the conductive member (5) is connected to a ground electrode layer (2C) consisting of the second conductive layer in each of divided element portions (A); and
filling gaps between the plurality of piezoelectric elements (2) arranged in the azimuth direction and between the plurality of divided element portions (A) in each of the piezoelectric elements (2) with a filler (4).
